# FASCICULE DE BREVET EUROPEEN

(11) **EP 1 006 937 B1**
(45) Date de publication et mention de la délivrance du brevet: **14.01.2004**
(21) Numéro de dépôt: 98906968.7
(22) Date de dépôt: 04.02.1998
(51) Int. Cl.: A61F 2/04

(54) **IMPLANT ARTIFICIEL DESTINE AU REMPLACEMENT DE L'APPAREIL EXCRETEUR URINAIRE CHEZ L'ETRE HUMAIN**
KÜNSTLICHES IMPLANTAT ZUM ERSETZEN DES MENSCHLICHEN HARNAUSSCHEIDUNGSORGANS
ARTIFICIAL IMPLANT INTENDED TO REPLACE THE HUMAN URINARY EXCRETORY ORGANS

(30) Priorité: 14.02.1997 FR 9701762
(43) Date de publication de la demande: 14.06.2000
(73) Titulaire: ASSISTANCE PUBLIQUE, HOPITAUX DE PARIS, 75100 Paris (FR)
(72) Inventeur: DESGRANDCHAMPS, François, F-75014 Paris (FR); LE DUC, Alain, F-92200 Neuilly sur Seine (FR)
(74) Mandataire: Vaillant, Jeanne
(86) Numéro de dépôt international: PCT/FR1998/000200
(87) Numéro de publication internationale: WO 1998/035633

(56) Documents cités:
- EP-A- 0 372 311
- WO-A-93/16659
- FR-A- 2 255 877

## Description

La présente invention concerne les implants artificiels destinés au remplacement de l'appareil excréteur urinaire chez l'être humain, homme ou femme.

Une des principales fonctions de l'appareil excréteur urinaire est le recueil et l'acheminement des fluides produits par le métabolisme rénal à travers les uretères vers la vessie, où lesdits liquides peuvent être emmagasinés pendant un certain temps. Lorsque les liquides ont atteint un certain niveau et exercent une certaine pression sur les parois de la vessie, ils sont acheminées vers la sortie à travers l'urètre, où le fonctionnement d'un muscle, le sphincter, autorise leur évacuation vers l'extérieur en agissant comme une valve.

Le remplacement de tout ou une partie de l'appareil excréteur urinaire est indiqué, par exemple, chez les malades ayant subi une cystectomie totale à cause d'un cancer de la vessie ou une résection pelvienne suite à un cancer rectal ou cervical. Autres indications pour ce type de remplacement sont les atteintes neurologiques vésicales ou par des cystites interstitielles, de la tuberculose, des radiations ou de la schistosomiase ou certaines malformations congénitales.

Seuls des implants naturels, utilisant du matériel biologique constitué par des tissus organiques divers, essentiellement des greffons intestinaux, ont pu être utilisés jusqu'à présent sans réaction de rejet à moyen terme chez l'homme.

Néanmoins, ces implants biologiques soulèvent de nombreux problèmes liés au reflux du liquide, aux infections, aux sécrétions des muqueuses, ou encore liés aux absorptions de produits urinaires sécrétés et des complications ostomales.

Ces inconvénients ont amené au développement d'implants artificiels présentant moins de risques que les implants biologiques naturels.

Plusieurs modèles de vessie, d'uretères, d'urètre ou de sphincter artificiels, comportant une pluralité de matériaux ont été développés séparément et à titre expérimental chez l'animal, mais, en dehors du sphincter artificiel, aucun d'entre eux n'a pu être utilisé avec succès pendant une longue période de temps chez l'être humain.

On a donc essayé de concevoir des prothèses entraînant moins de complications après implantation. Parmi les matériaux synthétiques testés, on connaît le vitellium, le Téflon^{(r)}, le polyvinyle, l'Ivalon^{(r)}, le Dacron^{(r)}, l'argent, le tantalum, ainsi que le polytétrafluoroéthylène expansé (Gore-Tex^{(r)}).

La plupart des matériaux utilisés n'ont pas abouti à des résultats satisfaisants, soit à cause du développement rapide d'infections liées à l'adhésion bactérienne sur les matériaux mis en oeuvre, soit à la formation de calculs, la déformation ou le décrochement de la prothèse, le reflux des liquides vers les reins, ou bien la rupture des éléments de l'implant au niveau des anastomoses, les jonctions entre les éléments artificiels et les éléments naturels.

Un matériau idéal pour un tel organe artificiel qui est destiné à être implanté à l'intérieur du corps humain, résiste à l'adhésion bactérienne. II est également biocompatible de façon à être bien toléré par les tissus environnants et à ne pas induire de réactions de rejet provoquant l'inflammation.

De plus, l'implant artificiel est de préférence rempli avec une faible augmentation de la pression intraluminale, de manière à conserver sa capacité de remplissage après vidange, et il ne se collapse pas.

Un problème additionnel à résoudre par la mise en oeuvre d'un implant artificiel destiné à remplacer des parties de l'appareil urinaire humain, est le raccourcissement de la durée de vie de l'implant in situ. Ce problème est essentiellement dû aux fuites de fluides, les urines présentant un fort caractère corrosif, autour des connexions anastomosées entre les différents éléments, à la cicatrisation du tissu autour de la prothèse et plus particulièrement aux dépôts des détritus contenus dans les urines sur les divers éléments de la prothèses qui conduisent à leur détérioration, voire leur obstruction.

Or, l'une des causes d'échec en ce qui concerne la durée du maintien en place des implants artificiels, dérive de l'utilisation des matériaux décrits ci-dessus, qui obligent à effectuer des remplacements périodiques de la prothèse implantée avec des interventions chirurgicales lourdes, ces interventions devant défaire les anastomoses formées pour permettre leur retrait.

Il est connu que la gomme de silicone évite l'incrustation de bactéries et constitue un matériau adéquat pour les implants artificiels qui doivent tenir longtemps en contact avec les fluides corporels.

Néanmoins, lorsque des vessies artificielles en silicone ont été implantées dans la cavité abdominale de chiens, avec des connecteurs en Téflon^{(r)}, comportant des filets en Marlex^{(r)} pour assurer la fonction fibreuse, des anastomoses étanches ont été obtenues mais des complications intestinales, suite aux réactions de rejet dues à l'intolérance de l'organisme au silicone, ont été développées par la suite.

Or un implant artificiel destiné à remplacer la vessie ne devrait provoquer ni rejet en tant que corps étranger, ni infection, ni encroûtement, ni reflux de liquides, ni dilatation des voies supérieures.

A ce jour, des remplacements partiels des éléments de l'appareil unitaire ont été divulgués, mais les dispositifs mis en oeuvre sont complexes et nécessitent des moyens de pompage hydrauliques sophistiqués pour le contrôle du flux descendant des urines et la commande de vidange de la vessie.

Ainsi le brevet US 5 370 690 de David M.Barret divulgue une vessie artificielle constituée d'une coque externe rigide en polysulfone et d'un réservoir interne flexible en silicone. Ces deux éléments délimitent entre eux un espace ouvert pouvant être rempli avec un liquide biocompatible à une pression prédéterminée. Cet espace intermédiaire est relié à un réservoir et a des moyens de pompage qui permettent, par l'exercice d'une pression, de vidanger le réservoir interne contenant les urines. Le dispositif divulgue également des uretères disposant de valves anti-retour pour empêcher la remontée des urines depuis la vessie vers les reins.

Par ailleurs, la publication *« Alloplastic replacement of the urinary bladder »* de Rohrman D. et al. in J.of Urology, Vol. 156, pages 2094-2097 (1996), décrit le remplacement de l'ensemble de l'appareil urinaire par un ensemble constitué par deux réservoirs latéraux implantés de façon sous-cutanée. Des problèmes de reflux des fluides, depuis les réservoirs constituant la vessie vers les reins, subsistent, obligeant à l'installation de deux valves anti-retour dans les deux cathéters reliant les réservoirs aux reins.

Il est connu du brevet français FR 2255877 de J.Chevallet et A.Sausse une prothèse vésicale implantable dans la cavité intra-abdominale, susceptible d'être raccordée aux uretères et à l'urètre d'un patient ou à des prothèses d'uretères et/ou d'urètre, et dépourvue de toute valve ou clapet artificiel, autorisant ainsi la commande manuelle par le patient lui-même.

Le dispositif présenté dans ce brevet comporte un réservoir constitué en une matière plastique souple, de préférence un élastomère vulcanisé, lui conférant la propriété de revenir à sa forme originale après avoir été déformé par la vidange des urines. Ce dispositif prévoit également le remplacement des conduits destinés aux connexions urétérales et/ou l'évacuation des urines à travers la paroi abdominale ou périnéale. Ces liaisons avec les éléments naturels restants sont effectuées par l'intermédiaire de manchons en matière textile colonisable visant à favoriser l'anastomose pour assurer le maintien en place de l'implant.

Ce dispositif est destiné à occuper l'emplacement de la vessie naturelle, de manière à ce que les muscles abdominaux contribuent à la vidanger en exerçant une pression limitée.. Pour ce faire, une intervention chirurgicale lourde est nécessaire pour l'insertion de l'implant et le remplacement de la vessie naturelle.

D.P. Griffith recense, outre l'implant artificiel de Barret, divers dispositifs dans sa publication: *« A prosthetic urinary bladder why not? »,* in Mayo Clin.Proc. vol.67 pp. 293-295 1992) et souligne les problèmes non résolus par les implants existants, à savoir les réactions inflammatoires provoquées par l'insertion de tuyaux artificiels dans les uretères et/ou l'urètre ainsi que les problèmes d'étanchéité qui se produisent notamment au niveau des anastomoses.

L'invention vise à résoudre les problèmes énoncés ci-dessus et, en particulier, à réaliser un implant ne nécessitant pas d'intervention chirurgicale lourde lors de sa mise en place ou lors du remplacement partiel d'un de ses éléments, tout en permettant un maintien en place de la prothèse à long terme. Pour cela, l'implant de la présente invention est un remplacement intégral de l'appareil urinaire afin d'éviter toute anastomose, et présente une structure particulière à double couche pour tous les organes de l'appareil urinaire. Cette structure permet le remplacement partiel de chacun de ses éléments et assure le maintien en place de la prothèse à long terme.

Plus précisément, l'invention a pour objet un implant artificiel, du type à remplacement intégral, comportant comme éléments artificiels de substitution aux organes de l'appareil urinaire humain, des éléments tubulaires pour les uretères et l'urètre, un réservoir pour la vessie, et un sphincter urétral artificiel. Les éléments artificiels sont structurés selon un agencement mixte, bi-couche, et sont liés entre eux par des connexions mécaniques. Ces éléments sont composés et conformés pour permettre leur passage entre un état aplati à vide et un état expansé. Les deux couches composant les éléments tubulaires sont solidaires et les deux couches composant le réservoir forment respectivement une coque externe, suffisamment flexible pour être déformable par pression manuelle, et une poche interne, souple et extractible de la coque.

La structure mixte selon l'invention est adaptée pour assurer l'étanchéité des ancrages aux reins et permettre d'éliminer les risques de développement d'infections et d'éliminer le risque de rejet de la part des tissus environnants. L'implant selon l'invention permet d'éviter en outre des interventions chirurgicales à répétition, et de diminuer par ailleurs les coûts inhérents à ce type d'interventions.

La couche interne reste en contact avec les fluides et possède une faible capacité d'adhérence pour les résidus, par exemple les bactéries, les protéines, les cristaux habituellement présents dans les urines et susceptibles de s'incruster sur les parois de l'implant, et un degré de souplesse élevé pour être extractible à travers un espace de type fente. La couche interne est par exemple composée de gomme de silicone.

La poche interne du réservoir peut ainsi être aisément extraite afin d'être remplacée par une nouvelle poche à travers les incisions utilisées en chirurgie légère, sans intervention sur les autres éléments de l'implant.

Outre ses qualités de flexibilité, le matériau de la couche externe de la structure bi-couche présente avantageusement un haut degré de biocompatibilité. Il est constitué par exemple à partir de polytétrafluoroéthylène expansé.

Selon d'autres caractéristiques préférées, des moyens manuels de pression directe avec un système à ressort, ou des moyens mécaniques ou électromagnétiques sont mis en oeuvre pour effectuer la vidange.

Les différents éléments composant l'implant artificiel de la présente invention sont de préférence reliés entre eux par des liaisons mécaniques. Avantageusement, ce type de liaison résout notamment les problèmes d'étanchéité des implants artificiels communément utilisés, qui se limitent au remplacement de l'un ou l'autre des composants naturels de l'appareil urinaire.

L'utilisation des connexions mécaniques entre les différents composants (uretères, vessie, urètre et sphincter) conjointement à l'ancrage direct des uretères au niveau des reins permet de maintenir l'ensemble en s'affranchissant de la formation d'anastomoses.

Grâce aux matériaux utilisés, l'implantation de l'appareil urinaire selon l'invention dans une couche superficielle permet la vidange de la vessie ainsi que l'ouverture du sphincter urétral par simple pression manuelle. De plus, l'implantation en sous-cutanée, à la place de l'emplacement dans la loge abdominale habituelle destinée à ce type d'implants ne nécessite qu'une intervention chirurgicale non invasive applicable à un grand nombre de patients.

D'autres caractéristiques et avantages de l'implant artificiel de la présente invention apparaîtront à la lecture de la description qui suit, accompagnée des figures annexes qui représentent respectivement :
- la figure 1, une vue générale en coupe d'un implant artificiel selon la présente invention ;
- la figure 2, une vue en coupe selon II-II du réservoir de l'implant selon la figure 1, destiné à remplacer la vessie ;
- la figure 3, une vue partiellement arrachée d'une liaison mécanique entre le réservoir et une uretère artificielle selon l'invention ;
- la figure 4, une vue latérale et partielle de l'implant selon l'invention ;
- la figure 5, une coupe présentant l'état du réservoir en cours de vidange,
- les figures 6a à 6c, trois exemples de sphincter urétral artificiel.

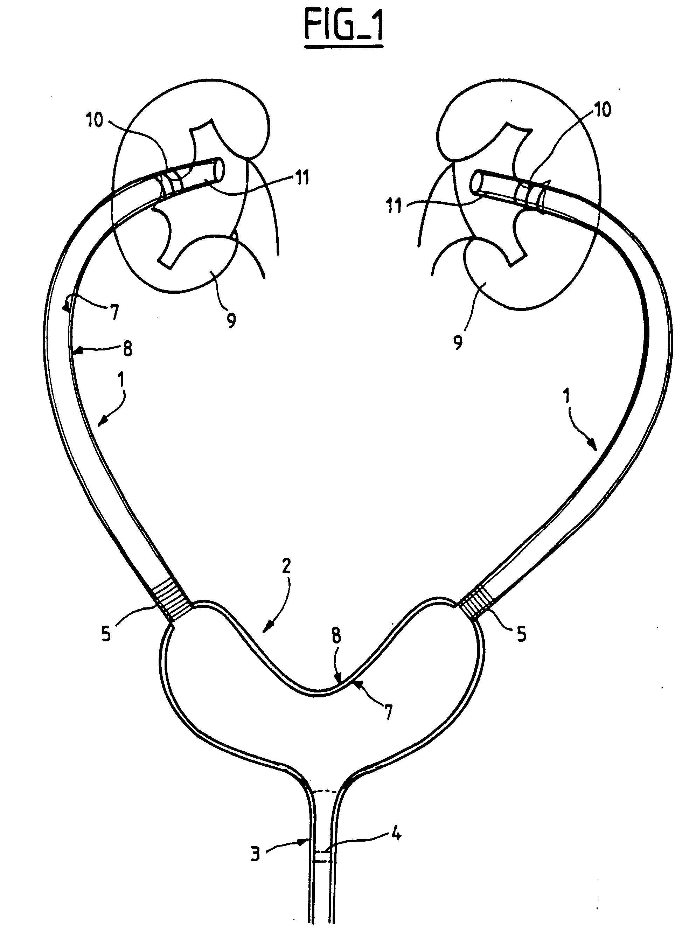

L'implant artificiel de la présente invention tel qu'illustré à la figure 1 remplace la totalité de l'appareil urinaire humain naturel, constitué principalement de la vessie, des uretères, de l'urètre et du sphincter urétral.

L'implant comporte principalement un réservoir 2, destiné à remplacer la vessie, auquel sont reliés, par des liaisons mécaniques 5, les éléments tubulaires 1, qui constituent les uretères artificiels de liaison aux reins 9. Le réservoir 2 se prolonge en un élément tubulaire 3 constitutif de l'urètre artificielle, celle-ci comprenant un sphincter artificiel 4.

Chacun des éléments 1 à 4 de l'implant de la présente invention est constitué par une structure mixte bi-couche, composée d'une couche interne 7 et d'une couche externe 8 en matériaux différents, respectivement en gomme de silicone et en polytétrafluoroéthylène expansé.

La couche interne empêche l'adhérence de résidus biologiques (protéines, bactéries, cristaux ou autres dépôts) habituellement présents dans les urines. D'autres polymères synthétiques ayant ces propriétés physiques vis-à-vis du matériel biologique sont connus de l'homme du métier, parmi eux on peut citer notamment le polyuréthane ou tout autre matériau biorésistant à l'incrustation dans l'urine.

La couche externe de cette structure mixte, destinée à être en contact avec les tissus vivants environnants, présente des caractéristiques de biocompatibilité optimales. Pour les éléments tubulaires 1 et 3, la couche externe est conformée sous forme d'une bandelette enroulée en spirale autour de la couche interne présentant une conformation tubulaire, ou sous la forme d'un tube directement extrudé autour de la couche interne. Pour les éléments tubulaires 1 et 3, les deux couches sont solidarisées.

La couche externe est composée d'un polymère qui permet de supprimer toute réaction de rejet de la part des organes vivants restant en contact permanent avec l'implant. Des polymères présentant ces caractéristiques sont également connus, et sont par exemple utilisés dans les prothèses cardio-vasculaires, comme le polytétrafluoroéthylène ou tout autre biomatériau poreux, tel que le polyuréthane, permettant la pénétration des tissus l'entourant, assurant ainsi l'étanchéité du montage.

L'implant artificiel de l'invention met en oeuvre pour réaliser les moyens de connexions mécaniques, des manchons de serrage, des bracelets à vis, anneaux ou bagues, pour relier entre eux les différents éléments remplaçant l'appareil urinaire. Ces connexions sont essentiellement réalisées avec la structure mixte solidarisée précédemment décrite.

Ces connexions mécaniques éliminent le besoin de formation d'anastomoses pour assurer le maintien de l'ensemble et garantissent par conséquent son étanchéité. Les risques de fuites sont ainsi avantageusement éliminés. De plus, le remplacement d'un des éléments de l'implant, en cas de défaillance de celui-ci, est plus simple, car il suffit de défaire une liaison mécanique et d'extraire la partie défaillante, au lieu de pratiquer une intervention chirurgicale pour défaire une anastomose.

Le maintien de l'ensemble de l'implant est assuré par la structure mixte constitutive de l'implant, adaptée pour former des moyens de fixation directe des éléments tubulaires 1 sur chaque parenchyme rénal 9. La couche externe 8 présente des plis 10, disposés en spirale ou en anneau, ces plis assurant avantageusement l'ancrage immédiat de l'implant sur le parenchyme rénale. Sur les extrémités distales 11 des uretères, la couche externe spiralée est retirée pour faciliter leur introduction dans les cavités rénales.

Les éléments de l'implant selon l'invention sont conformés et constitués pour pouvoir se présenter à plat à vide, et pouvoir passer entre cet état et un état expansé, et vice versa, lorsqu'il se remplit ou se vide. L'épaisseur des couches est adaptée par l'homme du métier pour que les matériaux sélectionnés, selon les critères de l'invention précédemment définis, soient suffisamment flexibles pour passer d'un état aplati à un état expansé. La structure est de plus initialement contrainte de manière à se présenter à vide sous la forme aplatie.

Ainsi, la structure mixte de l'implant artificiel de l'invention autorise son implantation sous la peau, à travers deux incisions para-iliaques, au moyen d'un ballonnet, selon une technique connue de l'homme du métier.

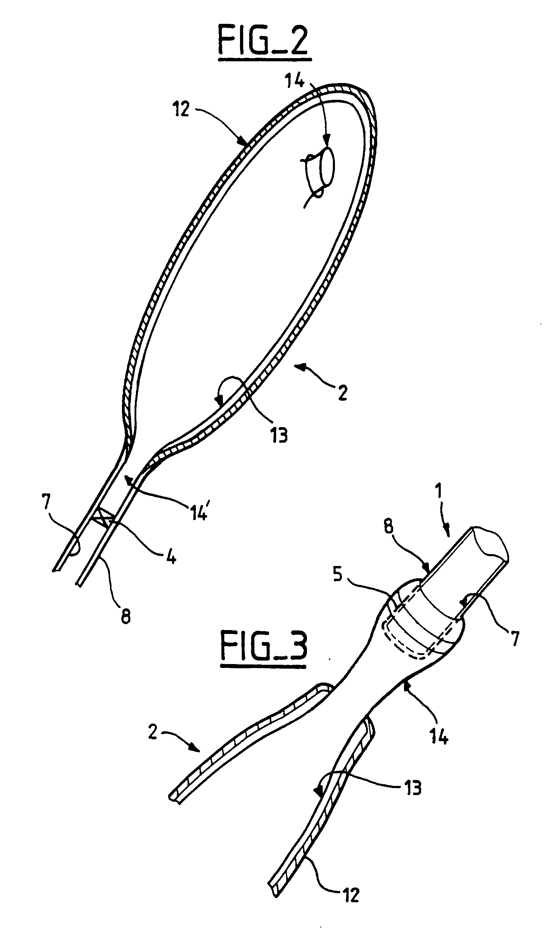

Le réservoir 2 constituant la partie de l'implant servant à remplacer la vessie naturelle est illustré sur la figure 2, qui est une vue en coupe selon II-II de la figure 1. Il est constitué par une coque externe 12 réalisée dans le même matériau biocompatible que la couche externe précédemment décrite, et par une poche interne souple 13, constituée par le même matériau que la couche interne précédemment décrite. La poche interne souple n'est pas solidaire de la coque externe : elle reste libre à l'intérieur de celle-ci, en épousant sa forme lorsqu'elle se remplit. Cette poche interne a une contenance maximale d'environ 500 millilitres.

Des protubérances 14 de la poche interne (une seule étant visible sur la figure, l'autre, symétrique, étant cachée), qui se prolongent au delà de la coque externe, servent à réaliser la liaison entre le réservoir 2 et les éléments tubulaires 1 constitutifs des uretères, au moyen des connexions mécaniques. Une autre protubérance 14' de la poche interne constitue le début de l'élément tubulaire constitutif de l'urètre. Ces protubérances sont réalisées par élongation ou par tout moyen connu de l'homme de l'art (moulage, collage, fusion,...) suivant le matériau constitutif utilisé.

Les éléments tubulaires constitutifs des uretères et de l'urètre sont également conformés avec la structure mixte décrite précédemment.

La figure 3 illustre plus particulièrement la liaison mécanique 5, entre le réservoir vésical 2 et les uretères 1, du type bague de serrage dans cet exemple de réalisation où l'uretère 1 est emmanchée dans la protubérance 14 de la poche intérieure 13 du réservoir 2. Ces liaisons mécaniques, qui peuvent se présenter sous d'autres formes, par exemple des bracelets à vis, facilitent le remplacement partiel de chacun des éléments constitutifs.

En particulier, ces liaisons permettent l'extraction et donc le remplacement aisé de la poche souple. Avantageusement, le matériau utilisé pour la couche interne et son épaisseur sont choisis pour que la poche soit suffisamment souple jusqu'à pouvoir se replier sur elle-même au moment de l'extraction. La reconstitution du réservoir vésical est ainsi facilitée et peut être réalisée avec une intervention chirurgicale simple, les autres éléments de l'appareil urinaire étant maintenus en place.

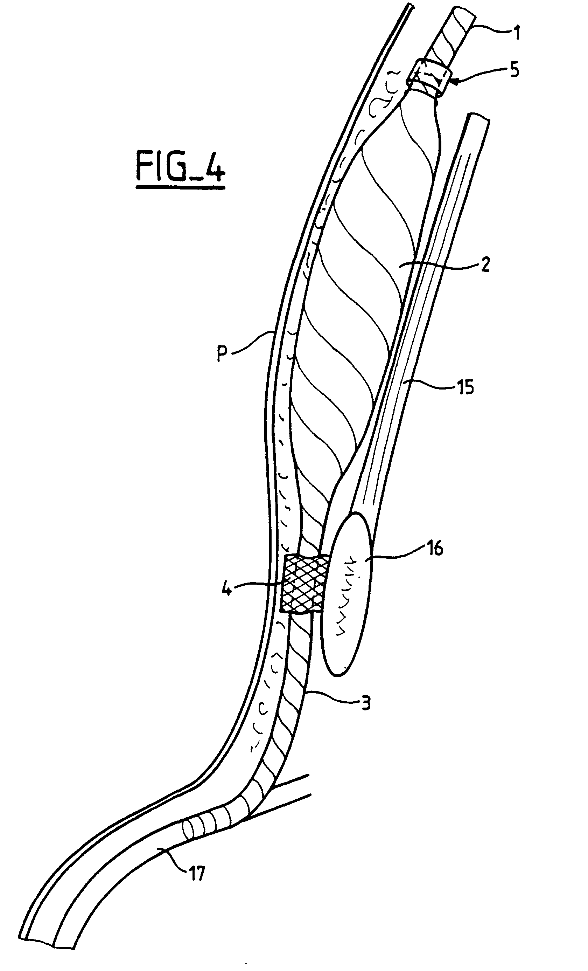

La figure 4 montre l'implantation en sous-cutané du réservoir 2 et de ses prolongements, selon une vue latérale partielle de la figure 1. Le réservoir 2 est disposé entre la peau P et le muscle pelvien 15. L'urètre 3 avec son système sphinctérien 4 passe devant le pubis 16 pour venir s'insérer dans le canal d'évacuation naturelle final 17. Les uretères 1 sont connectées au réservoir 2 par le connecteur 5 depuis l'autre extrémité du réservoir.

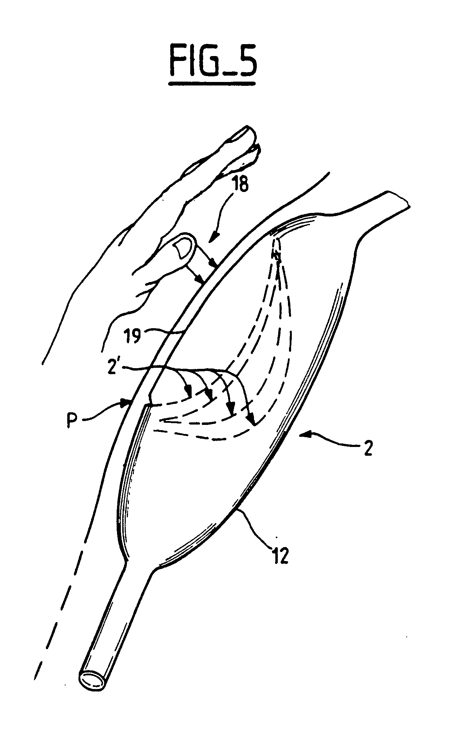

Par ailleurs, et tel qu'il est illustré sur la figure 5, la vidange des urines emmagasinées dans la poche interne du réservoir vésical 2 est effectué par une simple dépression manuelle 18 en appuyant sur la partie antérieure 19 de celui-ci, facilement accessible sous la peau P en raison de son implantation sous-cutanée. Le réservoir se déforme selon les lignes pointillées 2'. La coque externe 12, et en particulier la partie 19 de cette coque, possède avantageusement une épaisseur supérieure à celle de la couche externe des uretères et de l'urètre, de manière à augmenter sa résistance sans diminuer sensiblement sa flexibilité.

La pression exercée sur le réservoir 2 provoque l'ouverture du sphincter artificiel 4 permettant la sortie des urines vers l'extérieur, à travers l'urètre 3. Le choix du sphincter utilisé est guidé par sa capacité à pouvoir épouser au plus près l'éjection des urines.

Cette expulsion de l'urine vers l'extérieur, accompagnée de la fermeture simultanée du sphincter artificiel 4, crée une faible pression négative à l'intérieur de la poche 12 du réservoir 2. Cette faible pression négative, qui se maintient après la fermeture du sphincter 4, est suffisante pour assurer le drainage continu des fluides depuis les reins jusqu'au réservoir 2 à travers les uretères 1.

Avantageusement, la pression négative créée par la vidange du réservoir 2 empêche le reflux des urines vers le haut appareil urinaire, éliminant les risques d'hydronéphrose ou d'insuffisance rénale.

De ce fait, l'implant artificiel de la présente invention ne nécessite pas la mise en place de dispositifs complexes comme les valves anti-retour urétérovésicales ou urétrovésicales indispensables dans les implants destinés au remplacement total de l'appareil urinaire connus à ce jour. Par conséquent l'implant artificiel de la présente invention est moins onéreux et l'intervention chirurgicale, nécessaire à sa mise en place est simple, non invasive et peu traumatisante pour les patients.

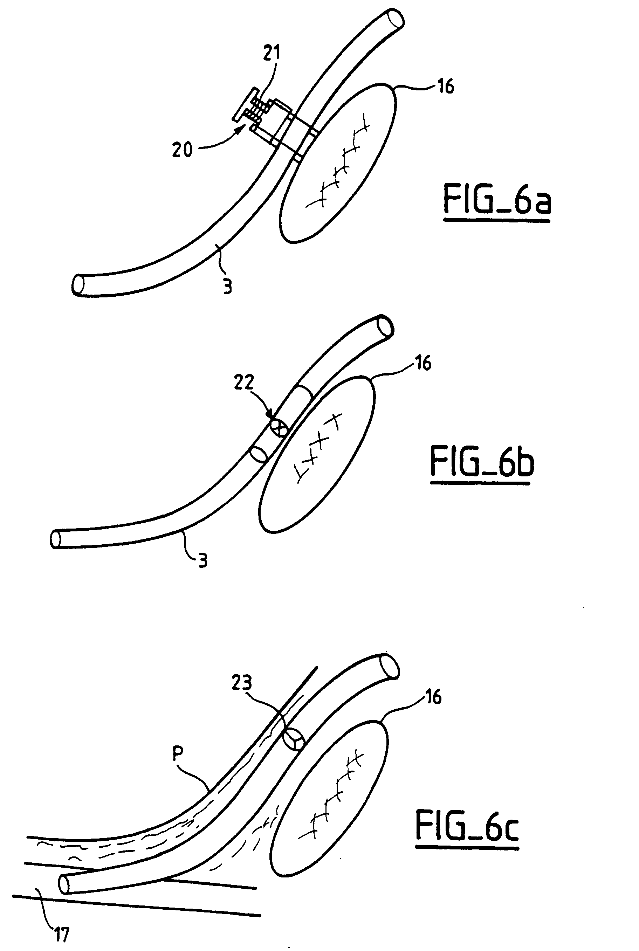

Les figures 6a à 6c illustrent différents exemples de réalisation du sphincter urétral artificiel, constitué par un système permettant la compression et décompression de l'urètre artificiel 3 devant le pubis 16 afin de permette la vidange du réservoir vésical.

Cette compression/décompression de l'urètre artificielle peut être activée, par exemple, par une valve manuelle 20 à ressort 21 (figure 6a), par l'intermédiaire d'une turbine électromagnétique 22 (figure 6b), ou par l'introduction d'une valve anti-retour 23 (figure 6c).

Ce système de compression est, comme les autres éléments de l'implant de l'invention, relié à l'ensemble par des connexions mécaniques non anastomosées, qui peuvent être défaites aisément pour faciliter leur remplacement partiel en cas de besoin.

L'invention n'est pas limitée aux exemples décrits et illustrés. Il est par exemple possible d'utiliser des formes variées pour le réservoir remplaçant la vessie, pour les uretères et l'urètre artificielles et les connexions mécaniques, la forme en spirale de la couche externe des tubulures pouvant par exemple être remplacée par tout type de forme. Par ailleurs, les connexions peuvent être réalisées sur une longueur adaptée aux matériaux utilisés et leur épaisseur; l'intervalle entre la poche interne et la coque du réservoir est également variable et adaptée.

## Revendications

1. Implant artificiel, du type à remplacement intégral, comportant comme éléments artificiels de substitution aux organes de l'appareil urinaire humain, des éléments tubulaires pour les uretères et l'urètre, un réservoir pour la vessie, et un sphincter urétral artificiel, **caractérisé en ce que** les éléments artificiels (1 à 3) sont structurés selon un agencement mixte, bi-couche (7,8), et sont liés entre eux par des connexions mécaniques (5), **en ce que** ces éléments sont composés et conformés pour permettre leur passage entre un état aplati à vide et un état expansé, **en ce que** les deux couches (7,8) composant les éléments tubulaires (1,3) sont solidaires et **en ce que** les deux couches (7,8) composant le réservoir (2) forment respectivement une coque externe (12), suffisamment flexible pour être déformable par pression manuelle, et une poche interne (13), souple et extractible de la coque.

2. Implant artificiel selon les revendications 1 dans lequel le matériau du réservoir (2) est déformable par application d'une pression manuelle (15) suffisante pour déclencher l'ouverture du sphincter urétral (4).

3. Implant artificiel selon l'une quelconque des revendications précédentes dans lequel les connexions mécaniques entre le réservoir (2) et les éléments tubulaires (5,6) sont supportées sur des protubérances (14,14') de la poche interne extractible (13).

4. Implant artificiel selon l'une quelconque des revendications précédentes dans lequel les connexions mécaniques (5, 6) entre ses éléments (1 à 3) sont choisis parmi des manchons de serrage, des bracelets à vis ou des bagues.

5. Implant artificiel selon l'une quelconque des revendications précédentes dans lequel la couche externe (8) des éléments tubulaires (1) destinés à être reliés à chacun des parenchymes rénaux (9) présente des plis extérieurs (10) en spirale ou en anneau permettant d'accrocher directement les parenchymes rénaux (9) pour assurer le maintien de l'ensemble, la couche externe (8) étant retirée sur les extrémités distales (11) de ces éléments tubulaires (1).

6. Implant artificiel selon l'une quelconque des revendications précédentes dans lequel règne une pression négative à l'intérieur du réservoir (2) remplaçant la vessie.

7. Implant selon l'une quelconque des revendications précédentes dans lequel le sphincter artificiel (4) est activé par une valve mécanique à ressort.

8. Implant selon l'une quelconque des revendications précédentes dans lequel le sphincter artificiel (4) est activé par une valve électromagnétique.

9. Implant selon l'une quelconque des revendications précédentes dans lequel la couche externe (8) est en biomatériau poreux.

10. Implant artificiel selon l'une quelconque des revendications précédentes dans lequel la couche interne (7) est en biomatériau résistant à l'incrustation dans l'urine.

11. Implant artificiel selon l'une quelconque des revendications 1 à 10 précédentes, dans lequel, pour les éléments tubulaires (1, 3), la couche externe (8) est conformée sous la forme d'un tube directement extrudé autour de la couche interne (7).

## Patentansprüche

1. Künstliches Implantat, von der Art eines integralen Ersatzes, umfassend als künstliche Elemente zur Substitution von Organen des menschlichen Harnausscheidungsapparats, rohrförmige Elemente für die Harnleiter und die Harnröhre, ein Reservoir für die Blase und einen künstlichen Harnröhrenschließmuskel, **dadurch gekennzeichnet, dass** die künstlichen Elemente (1 bis 3) in einer gemischten Anordnung strukturiert, zweilagig (7, 8) und miteinander durch mechanische Verbindungen (5) verbunden sind, dadurch, dass die Elemente so zusammengestellt und angepasst sind, dass sie ihren Übergang zwischen einem leeren flachen Zustand und einem ausgedehnten Zustand erlauben, dadurch, dass die die rohrförmigen Elemente (1, 3) bildenden beiden Schichten (7, 8) verbunden sind und dadurch, dass die das Reservoir (2) bildenden beiden Schichten (7, 8) entsprechend eine äußere Hülle (12) bilden, die ausreichend flexibel ist, so dass sie unter Druck von Hand verformbar ist, und einen inneren Beutel (13), der weich und aus der Hülle herausnehmbar ist.

2. Künstliches Implantat nach Anspruch 1, bei dem das Material des Reservoirs (2) durch Anwendung eines Druckes mit der Hand (15) verformbar ist, was ausreicht, um die Öffnung des Harnröhrenschließmuskels (4) auszulösen.

3. Künstliches Implantat nach einem der vorhergehenden Ansprüche, bei dem die mechanischen Verbindungen zwischen dem Reservoir (2) und den rohrförmigen Elementen (5, 6) auf Vorsprüngen (14, 14') des herausnehmbaren inneren Beutels (13) gestützt sind.

4. Künstliches Implantat nach einem der vorhergehenden Ansprüche, bei dem die mechanischen Verbindungen (5, 6) zwischen den Elementen (1 bis 3) aus Spannhülsen, Schraubschellen oder Ringen ausgewählt sind.

5. Künstliches Implantat nach einem der vorhergehenden Ansprüche, bei dem die äußere Schicht (8) der rohrförmigen Elemente (1), die zum Verbinden mit jedem der Nierenparenchyme (9) vorgesehen sind, spiralförmige oder ringförmige äußere Falten (10) aufweist, was direktes Anhaften der Nierenparenchyme (9) erlaubt, um den Halt der Anordnung zu gewährleisten, wobei die äußere Schicht (8) auf die distalen Enden (11) dieser röhrförmigen Elemente (1) zurückgezogen ist.

6. Künstliches Implantat nach einem der vorhergehenden Ansprüche, bei dem im Inneren des die Blase ersetzenden Reservoirs (2) ein negativer Druck herrscht.

7. Künstliches Implantat nach einem der vorhergehenden Ansprüche, bei dem der künstliche Schließmuskel (4) durch ein mechanisches Federventil aktiviert wird.

8. Künstliches Implantat nach einem der vorhergehenden Ansprüche, bei dem der künstliche Schließmuskel (4) durch ein elektromagnetisches Ventil aktiviert wird.

9. Künstliches Implantat nach einem der vorhergehenden Ansprüche, bei dem die äußere Schicht (8) ein poröses Biomaterial ist.

10. Künstliches Implantat nach einem der vorhergehenden Ansprüche, bei dem die innere Schicht (7) ein gegen Ablagerung im Urin beständiges Biomaterial ist.

11. Künstliches Implantat nach einem der vorhergehenden Ansprüche, bei dem die aüßere Schicht (8) der rohrförmigen Elemente (1, 3) rohrförmig direkt um der inneren Schicht (7) extrudiert ist.

## Claims

1. An artificial implant of the integral replacement type comprising, as artificial elements for substituting the organs of the human urinary system, tubular elements for the ureters and urethra, a reservoir for the bladder, and an artificial urethral sphincter, **characterized in that** the artificial elements (1 to 3) are structured as a mixed double-layered arrangement (7, 8) and are connected together by mechanical connections (5), and **in that** the composition and form of these elements are such as to permit their passage between a flattened state when empty and an expanded state, **in that** the two layers (7, 8) comprising the tubular elements (1, 3) are integral and **in that** the two layers (7, 8) comprising the reservoir (2) respectively form an outer shell (12), which is sufficiently flexible to be deformable by manual pressure, and in inner pouch (13), which is flexible and which can be extracted from the shell.

2. An artificial implant according to claim 1, in which the material of the reservoir (2) is deformable on application of a manual pressure (15) which is sufficient to cause opening of the urethral sphincter (4) .

3. An artificial implant according to any one of the preceding claims, in which the mechanical connections between the reservoir (2) and the tubular elements (5, 6) are supported on protuberances (14, 14') from the extractable inner pouch (13).

4. An artificial implant according to any one of the preceding claims, in which the mechanical connections (5, 6) between its elements (1 to 3) are selected from turnbuckles, worm drive clips and rings.

5. An artificial implant according to any one of the preceding claims, in which the outer layer (8) of the tubular elements (1) intended to be connected to each of the renal parenchymas (9) have external folds (10) in a spiral or a ring which permit to attach directly to the renal parenchymas (9) and to hold the assembly, the outer layer (8) being taken back from the distal ends (11) of the tubular elements (1).

6. An artificial implant according to any one of the preceding claims, wherein there is a negative pressure in reservoir (2) replacing the bladder.

7. An artificial implant according to any one of the preceding claims, in which the artificial sphincter (4) is activated by a mechanical spring valve.

8. An artificial implant according to any one of the preceding claims, in which the artificial sphincter (4) is activated by an electromagnetic valve.

9. An artificial implant according to any one of the preceding claims, in which the outer layer (8) is of porous biomaterial.

10. An artificial implant according to any one of the preceding claims, in which the inner layer (7) is of biomaterial which is resistant to encrustation by urine.

11. An artificial implant according to any one of the preceding claims, in which the outer layer (8) of the tubular elements (1, 3) is conformed as a tube which has been extruded directly around the inner layer (7).
